**Europäisches Patentamt**

**European Patent Office**

**Office européen des brevets**

(19)

(11) Veröffentlichungsnummer: **0 073 342**
**B1**

(12)
# EUROPÄISCHE PATENTSCHRIFT

(45) Veröffentlichungstag der Patentschrift:
**04.12.85**

(21) Anmeldenummer: **82106712.1**

(22) Anmeldetag: **24.07.82**

(51) Int. Cl.⁴: **B 01 J 31/40,** C 07 C 51/54

(54) Verfahren zur Reinigung und Rückgewinnung der bei der Carbonylierung von Methylacetat und/oder Dimethylether anfallenden, verunreinigten Katalysatorlösung.

(30) Priorität: **31.08.81 DE 3134350**

(43) Veröffentlichungstag der Anmeldung:
**09.03.83 Patentblatt 83/10**

(45) Bekanntmachung des Hinweises auf die Patenterteilung:
**04.12.85 Patentblatt 85/49**

(84) Benannte Vertragsstaaten:
**AT BE CH DE FR GB IT LI NL**

(56) Entgegenhaltungen:
**EP - A - 0 026 280**
**FR - A - 2 232 364**
**US - A - 2 790 007**
**US - A - 3 065 242**
**US - A - 3 821 311**

(73) Patentinhaber: **HOECHST AKTIENGESELLSCHAFT,**
**Postfach 80 03 20, D-6230 Frankfurt am Main 80 (DE)**

(72) Erfinder: **Erpenbach, Heinz, Dr., Oberbuschweg 22,**
**D-5000 Köln 50 (DE)**
Erfinder: **Gehrmann, Klaus, Dr.,**
**Geschwister-Scholl-Strasse 14, D-5042 Erftstadt (DE)**
Erfinder: **Lork, Winfried,**
**Siegfried-von-Westerburg-Strasse 14, D-5042 Erftstadt**
**(DE)**
Erfinder: **Prinz, Peter, von Geyr-Ring 104, D-5030 Hürth**
**(DE)**

**Beschreibung**

Die Erfindung betrifft ein Verfahren zur Reinigung und Rückgewinnung der bei der Carbonylierung von Methylacetat und/oder Dimethylether anfallenden, verunreinigten Katalysatorlösung, welche Carbonylkomplexe von Edelmetallen der Gruppe VIII des Periodensystems der Elemente, quaternäre heterocyclische aromatische Stickstoffverbindungen oder quaternäre Organophosphorverbindungen als organische Promotoren, undestillierbare organische Verunreinigungen sowie Essigsäure, Essigsäureanhydrid und Ethylidendiacetat enthält.

Die Rückgewinnung von Rhodium- bzw. Rhodiumcarbonylkomplexen aus mit Rückständen verunreinigten Katalysatorsystemen oder Destillationssümpfen ist im wesentlichen bei Hydroformylierungsverfahren bekannt. So wird in der DE-C-1 290 535 der rhodiumhaltige Sumpf der Hydroformylierung bei erhöhter Temperatur mit wäßrigen organischen Säuren behandelt, wobei der Rhodiumkomplex in Lösung geht und so von der organischen Phase abgetrennt wird. Dabei wird eine Rhodiumausbeute von 82 bis 94% erzielt.

In der DE-B-1 295 537 wird der rhodiumhaltige Reaktionssumpf mit Wasserdampf von 2-31 bar und 100—250°C behandelt. Durch diese Maßnahme wird der Katalysator zersetzt und der anfallende Rhodiumschlamm, der als solcher für den Carbonylierungsprozeß nicht wieder einsetzbar ist, mittels Druckfiltration gewonnen.

Andere Möglichkeiten der Rhodiumwiedergewinnung bzw. der Katalysatorregenerierung beschreiben die DE-A-2 448 005 und DE-A-2 614 799. Hierbei werden die verunreinigten Destillationssümpfe aus der Hydroformulierung zur Zerstörung der Rhodium- bzw. Iridiumcarbonylkomplexe und Abbau der Rückstände zunächst einer Behandlung mit sauerstoffhaltigen Mineralsäuren und Peroxiden unterzogen. Nach Zersetzen der überschüssigen Peroxide werden die wäßrigen Metallsalzlösungen unterschiedlich aufgearbeitet. Gemäß DE-A-2 448 005 werden die Metalle nach Zusatz eines wasserlöslichen organischen Lösemittels, einer Halogenwasserstoffsäure oder eines Alkalihalogenids sowie eines tertiären Phosphins zur wäßrigen Rhodium- bzw. Iridiumsalzlösung bei 0—150°C und 1—250 bar mit Kohlenmonoxid oder auch zusätzlich unter hydrierenden Bedingungen als Rhodium bzw. Iridiumcarbonylkomplexe aus der Lösung abgeschieden. Gemäß DE-A-2 614 799 wird aus der wäßrigen Lösung das Rhodium$^{+++}$ an einem Kationenaustauscher absorbiert, danach mit Salzsäure desorbiert. Aus der salzsauren Lösung wird nach Zusatz von tertiären Phospinen und Behandeln mit CO und ggf. Wasserstoff das Rhodium als Carbonylkomplex ausgefällt.

Für die Reinigung und Rückgewinnung der Edelmetallcarbonylkomplexe (Rh, Ir, Pd, Ru) und ihrer Promotoren aus der bei der Carbonylierung von Methylacetat oder Dimethylether (z. B. gemäß den Verfahren der DE-A-2 450 965, 2 836 084, 2 939 839 oder 2 941 232) anfallenden verunreinigten Katalysatorlösung sind die beschriebenen Methoden nicht anwendbar. Die für die genannte Carbonylierungsreaktion verwendete Katalysatorlösung besteht aus 0,1—10 Gew.-% Edelmetallcarbonylkomplex, 40—70 Gew.-% organischen Promotoren, 1—10 Gew.-% undestillierbaren organischen Verunreinigungen und 20—40 Gew.-% Essigsäure, Essigsäureanhydrid und Ethylidendiacetat. Diese Zusammensetzung macht deutlich, daß die Katalysatorlösung bis zu 80 Gew.-% undestillierbare Substanzen enthalten kann. Ein oxidativer Abbau des Edelmetallcarbonylkomplexes nach Destillation der flüchtigen Bestandteile würde gleichzeitig die Zerstörung des gesamten organischen Promotoranteils bedeuten, wodurch der Nutzen einer Katalysatoraufarbeitung von vornherein infrage gestellt wäre.

Auch eine Behandlung des Rückstandes mit wäßrigen organischen Säuren ist von Nachteil, da hierbei neben dem Edelmetallcarbonylkomplex auch der größe Teil der undestillierbaren organischen Verunreinigungen in Lösung geht und nicht vom Katalysator und Promotor abgetrennt werden kann.

Die Zersetzung des Katalysators mit Wasserdampf bei höherer Temperatur führt zu Edelmetall in elementarer Form, das nur unter hohem Aufwand wieder in den aktiven Katalysatorkomplex zurückgeführt werden kann. Darüber hinaus fällt aber gleichzeitig auch der in Wasser unlösliche organische Rückstand an, der vom elementaren Edelmetall nicht ohne weiteres abzutrennen ist.

Die vorliegende Erfindung umgeht die aufgezeigten Nachteile und beschreibt ein Verfahren, das es mit Hilfe destillativer und extraktiver Methoden ermöglicht, die bei der Carbonylierung von Methylacetat und/oder Dimethylether eingesetzte und im Laufe des Prozesses verunreinigte Katalysatorlösung so aufzuarbeiten, daß unter Ausschleusung der undestillierbaren organischen Verunreinigungen sowohl der Edelmetall-Carbonyl-Komplex als auch seine Promotoren dem Katalysatorkreislauf wieder zugeführt werden können. Daneben wird im Vergleich zu den bekannten Aufarbeitungsverfahren durch den Kreislauf der zur Aufarbeitung eingesetzten Extraktionsmittel eine größere Umweltbelastung durch Abfallstoffe vermieden. Lediglich die im Prozeß und durch den Reaktionsablauf gebildeten undestillierbaren organischen Verunreinigungen werden ausgeschleust und müssen entsprechend dem Stand der Technik, z. B. durch Verbrennung, beseitigt werden.

Im einzelnen ist die Erfindung nunmehr dadurch gekennzeichnet, daß man die flüchtigen Bestandteile aus der Katalysatorlösung abdestilliert und den verbleibenden festen Destillationsrückstand mit Wasser behandelt, wobei der Edelmetall-Carbonyl-Komplex zusammen mit den organischen Verunreinigungen ausgefällt wird, während der organische Promotor in Lösung geht, daß man den ausgefällten verunreinigten Edelmetall-Carbonyl-Komplex abfiltriert und durch Extraktion mit aliphatischen Ethern von den organischen Verunreinigungen befreit, daß man den organischen Promotor durch Abdampfen des Wassers oder durch Extraktion mit Halogenkohlenwasserstoffen und Abdampfen derselben zurückgewinnt und daß man den gereinigten Edelmetall-Carbonyl-Komplex sowie den zurückgewonnenen organischen Promotor in die Carbonylierungsreaktion zurückführt.

Das Verfahren der Erfindung kann weiterhin bevorzugt und wahlweise dadurch gekennzeichnet sein, daß

a)  man die Behandlung des Destillationsrückstands mit Wasser bei 40—80°C vornimmt;
b)  man je Gewichtsteil Destillationsrückstand 10 bis 100 Gewichtsteile Wasser einsetzt,
c)  man den abfiltrierten Edelmetall-Carbonyl-Komplex mit Diethyl- oder Diisopropylether extrahiert,
d)  man quaternäre Organophosphorverbindungen aus ihrer wäßrigen Lösung durch Extraktion mit Methylenchlorid oder Chloroform zurückgewinnt,
e)  die Katalysatorlösung zusätzlich Verbindungen carbonylbildender Nicht-Edelmetalle als anorganische Promotoren enthält, welche bei der Behandlung des festen Destillationsrückstandes mit Wasser

    1.  zusammen mit dem organisch verunreinigten Edelmetall-Carbonyl-Komplex ausgefällt, abfiltriert, durch Extraktion mit Ether gereinigt und zusammen mit dem Edelmetall-Carbonyl-Komplex in die Carbonylierungsreaktion zurückgeführt werden und/oder
    2.  zusammen mit dem organischen Promotor in Lösung gehen und durch Abdampfen des Wassers oder Extraktion mit Halogenkohlenwasserstoffen und Abdampfen derselben zurückgewonnen und zusammen mit dem organischen Promotor in die Carbonylierungsreaktion zurückgeführt werden.

Zur Herkunft der verunreinigten Katalysatorlösung ist zu sagen, daß die aus einem Carbonylierungsreaktor abfließende Reaktionsmischung destillativ in die gewünschten Endprodukte, besonders Essigsäureanhydrid, Essigsäure und/oder Ethylidendiacetat, und nichtumgesetzte, im Kreislauf geführte Ausgangsstoffe einerseits sowie die als Sumpfprodukt anfallende Katalysatorlösung andererseits aufgetrennt wird. Ein Teilstrom dieser mit der Zeit verunreinigten Katalysatorlösung wird aus dem Kreislauf der Katalysatorlösung entnommen und erfindungsgemäß destillativ, vorzugsweise bei 100—120°C und 1—100 mbar, von den flüchtigen Bestandteilen wie Essigsäure, Essigsäureanhydrid und Ethylidendiacetat befreit.

Die verunreinigten Katalysatorlösungen enthalten als Edelmetalle im allgemeinen Rhodium, Iridium, Palladium und/oder Ruthenium, die als Carbonylkomplexe wie z. B.

$$[CH_3P(C_4H_9)_3]_2Rh(CO)I_5 \text{ oder } CH_3P(C_4H_9)_3Rh(CO)_2I_2$$

vorliegen. Die Katalysatorlösungen enthalten ferner als organische Promotoren bevorzugt einen oder mehrere der folgenden heterocyclischen aromatischen Stickstoffverbindungen oder Organophosphorverbindungen:

1.  N-Methylpyridiniumjodid, N,N-Dimethylimidazoliumjodid, N-Methyl-3-picoliniumjodid, N-Methyl-2,4-lutidinium-jodid, N-Methyl-3,4-lutidiniumjodid, N-Methyl-chinoliniumjodid.
2.  Tri-n-butyl-methyl-phosphoniumjodid, Trioctyl-methyl-phosphoniumjodid, Trilauryl-methyl-phosphoniumjodid, Triphenyl-methyl-phosphoniumjodid.

Schließlich kann die Katalysatorlösung als anorganische Promotoren Verbindungen der carbonylbildenden Nicht-Edelmetalle Ce, Ti, Zr, Hf, Ge, Sn, Pb, V, Nb, Ta, As, Sb, Bi, Cr, Mo, W, Mn, Re, Fe, Co, Ni enthalten.

Der verbleibende Destillationsrückstand wird sodann zweckmäßig unter Rühren in Wasser eingetragen und anschließend auf z. B. 70°C erwärmt. Der Anteil des Destillationsrückstandes an organischem Promotor löst sich dabei in der Wasserphase auf, während der Edelmetallcarbonylkomplex mit den bei der Reaktion gebildeten undestillierbaren organischen Verunreinigungen ungelöst bleibt. Der in der Wasserphase unlösliche Rückstand wird abfiltriert und einer Etherextraktion unterworfen. Mit Hilfe der Ether gelingt es, aus dem wasserunlöslichen Rückstand die organischen Verunreinigungen herauszulösen, so daß als Extraktionsrückstand der gereinigte Edelmetallcarbonylkomplex erhalten wird, der sofort dem Prozeß wieder zugeführt werden kann. Die in der Etherphase gelösten undestillierbaren organischen Verunreinigungen werden nach Abtreiben des Ethers in einer Verbrennungsanlage vernichtet. Der in der Wasserphase gelöste Promotor wird nach Abdampfen des Wassers in reiner Form gewonnen und wieder in die Reaktion eingesetzt. Sollen — falls quaternäre Organophosphorverbindungen als organische Promotoren vorliegen — die Verdampfungskosten des Wassers eingespart werden, so kann der in der Wasserphase gelöste organische Promotor auch mit Halogenkohlenwasserstoffen extrahiert werden. Nach Verdampfen des Extraktionsmittels hinterbleibt der reine organische Promotor, der dem Katalysatorkreislauf wieder zugeführt wird. Aus dem Wasserraffinat wird durch Abstreifen der gelöste Halogenkohlenwasserstoff entfernt. Danach können die Wasserphase, Halogenkohlenwasserstoffe und Ether wieder für einen erneuten Einsatz verwendet werden. Das

3

Verfahren der Erfindung kann sowohl in kontinuierlicher als auch diskontinuierlicher Betriebsweise durchgeführt werden.

## Beispiel 1

Dem aus Rhodiumcarbonylkomplex ($L_2Rh(CO)I_5$; L = Ligand), Tri-n-butylmethylphosphoniumjodid als organischem Promotor, Essigsäureanhydrid, Essigsäure sowie Ethylidendiacetat und organischen Verunreinigungen bestehenden Katalysatorkreislauf der Methylacetatcarbonylierung werden 250 g Katalysatorlösung entnommen und unter vermindertem Druck von etwa 2 mbar sowie einer Blasentemperatur bis zu 120° C von den destillierbaren Anteilen befreit. Dabei werden 81,5 g (32,6% Gew.-%) Destillat (25,8 Gew.-% Essigsäure, 73,8 Gew.-% Essigsäureanhydrid und 0,4 Gew.-% Ethylidendiacetat) und 168,5 g Destillationsrückstand mit 0,433 g Rhodium und 145,71 g Tri-n-butylmethylphosphoniumjodid (= 2 Gew.-% Rh-carbonyl-komplex und 58,3 Gew.-% TBMPJ, ber. auf 250 g Katalysatorlösung) erhalten. Der Destillationsrückstand wird zermörsert und unter kräftigem Rühren in 5000 ml Wasser von 20—25° C eingetragen. Nach 30 Minuten wird die Suspension unter weiterem Rühren auf 60—70° C erwärmt. Nach insgesamt 1,5 Stunden wird der Rückstand filtriert, mit Wasser nachgewaschen und anschließend 1,5 Stunden bei 120° C/2 mbar getrocknet. Die Auswaage ergibt 22,75 g, ihre Analyse 0,432 g Rhodium. Der wasserunlösliche Rückstand wird zur Entfernung der undestillierbaren organischen Verunreinigungen im Soxhlet-Apparat mit 500 ml Diisopropylether bei Siedetemperatur des Ethers extrahiert. Nach drei- bis fünfstündiger Extraktion und anschließender Trocknung wird der im Ether unlösliche Rückstand mit 6,75 g ermittelt. Sein Rhodiumgehalt beträgt 0,432 g.

Der etherunlösliche Rückstand enthält damit nahezu den gesamten in den Reinigungsprozeß eingesetzten Rhodiumcarbonylkomplex, der als solcher wieder in die Reaktion eingesetzt werden kann. Die Diisopropyletherphase wird eingedampft, wobei 16 g undestillierbare organische Verunreinigungen (= 6,4 Gew.-%, ber. auf 250 g Katalysatorlösung) mit einem Rhodiumgehalt von 0,01 Gew.-% im Kolben zurückbleiben, woraus sich für den Reinigungsprozeß eine Rhodiumausbeute von 99,6% errechnet. Der abdestillierte Ether wird zur erneuten Reinigung wieder verwendet.

Die aus der Filtration stammende Wasserphase wird gleichfalls unter vermindertem Druck aufgearbeitet, wobei 145,5 g Tri-n-Butylmethylphosphoniumjodid als Destillationsrückstand entsprechend 99,8% Ausbeute erhalten werden. Sowohl die P/J-Analyse mit 9 Gew.-% P und 36,9 Gew.-% Jod, die IR-Analyse und auch der Schmelzpunkt von 140° C zeigen den hohen Reinheitsgrad des zurückgewonnenen Salzes an. Rhodium kann an dieser Stelle durch Analyse nicht nachgewiesen werden. Das organische Promotorsalz wird als solches in den Prozeß wieder eingesetzt. Die abgedampfte Wasserphase findet in einer erneuten Extraktion Wiederverwendung.

## Beispiel 2

Dem aus Essigsäureanhydrid, Essigsäure, Ethylidendiacetat, Rhodiumcarbonylkomplex ($LRh(CO)_2I_2$; L = Ligand), Tri-n-butylmethylphosphoniumjodid und undestillierbaren organischen Verunreinigungen bestehenden Katalysatorkreislauf der Dimethylethercarbonylierung werden 250 g Katalysatorlösung entnommen. Unter vermindertem Druck von 2 mbar und einer Blasentemperatur bis zu 120° C werden die destillierbaren Anteile abgetrennt. Dabei werden von der eingesetzten Katalysatorlösung 72 g (28,8 Gew.-%) Destillat (31 Gew.-% Essigsäure, 68,6 Gew.-% Essigsäureanhydrid und 0,4 Gew.-% Ethylidendiacetat) und 178 g Destillationsrückstand mit 1,718 g Rhodium und 156,3 g Tri-n-butylmethylphosphoniumjodid (= 4,35 Gew.-% Rh-carbonyl-komplex und 62,5 Gew.-% TBMPJ, ber. auf 250 g Katalysatorlösung) erhalten. Der Destillationsrückstand wird vermahlen und anschließend unter Rühren in 6000 ml Wasser von 20° C eingegeben. Nach 30 Minuten wird die Suspension unter weiterem Rühren auf 60—70° C erwärmt. Nach einer Gesamtzeit von 1,5 Stunden wird der verbleibende Rückstand filtriert, mit Wasser nachgewaschen und anschließend bei 120° C und 2 mbar Druck getrocknet. Die Auswaage ergibt 21,75 g, ihre Analyse 1,718 g Rhodium. Dieser Rückstand wird zur Entfernung von undestillierbaren organischen Verunreinigungen im Soxhlet-Apparat mit 500 ml Diethylether beim Siedepunkt des Ethers extrahiert. Nach drei- bis fünfstündiger Extraktion und anschließender Trocknung wird ein etherunlöslicher Rückstand von 11,46 g erhalten. Der Rhodiumgehalt beträgt 1,71 g, entsprechend einer Ausbeute von 99,5%. Der etherunlösliche Rückstand enthält nahezu den gesamten in den Reinigungsprozeß eingesetzten Rhodiumcarbonylkomplex, der wieder in die Reaktion zurückgeführt wird.

Die Diethyletherphase wird eingedampft, wobei 10,29 g undestillierbare organische Verunreinigungen (= 4,12 Gew.-%, ber. auf 250 g Katalysatorlösung) im Kolben verbleiben. Laut Analyse besitzt dieser Rückstand noch einen Rhodiumgehalt von 0,09 Gew.-%. Der abdestillierte Diethylether kann für nachfolgende Versuche Wiederverwendung finden.

Die bei der Filtration anfallende Wasserphase wird zur Rückgewinnung des organischen Promotors sechsmal mit je 250 ml Chloroform ausgeschüttelt. Aus der Chloroformphase werden nach Abdampfen des Chloroforms 156 g Tri-n-butylmethylphosphoniumjodid entsprechend einer Ausbeute von 99,8% erhalten. P/J-Analyse mit 9 Gew.-% Phosphor und 36,9 Gew.-% Jod, IR-Analyse und Schmelzpunkt lassen auch hier den hohen Reinheitsgrad des zurückgewonnenen organischen Promotorsalzes erkennen. Die extrahierte Wasserphase steht nach Abstreifen des gelösten Chloroforms dem erneuten Einsatz zur Verfügung. Auch das Chloroform wird für weitere Extraktionen des organischen Promotors wieder eingesetzt.

Selbstverständlich kann die Extraktion des in der Wasserphase gelösten Promotors anstelle des Ausschüttelns mit Chloroform auch durch eine kontinuierlich geführte Extraktion in einer Mehrbodenkolonne durchgeführt werden.

## Beispiel 3

Dem aus Rhodiumcarbonylkomplex ($L_2Rh(CO)I_5$; L = Ligand), N,N-Dimethylimidazoliumjodid, undestillierbaren organischen Verunreinigungen, Essigsäure, Essigsäureanhydrid und Ethylidendiacetat bestehenden Katalysatorkreislauf der Methylacetatcarbonylierung werden 250 g Katalysatorlösung entnommen. Unter vermindertem Druck von 2 mbar und einer Blasentemperatur bis zu 120° C werden die destillierbaren Anteile entfernt. Dabei werden von der eingesetzten Katalysatorlösung 64 g (25,6 Gew.-%) Destillat (29,3 Gew.-% Essigsäure, 70,3 Gew.-% Essigsäureanhydrid und 0,4 Gew.-% Ethylidendiacetat) sowie 186 g Destillationsrückstand mit 0,525 g Rhodium und 161 g N,N-Dimethylimidazoliumjodid (= 1,96 Gew.-% Rh-carbonyl-komplex und 64,4 Gew.-% DMIJ, ber. auf 250 g Katalysatorlösung) erhalten. Der Destillationsrückstand wird zerkleinert und anschließend unter Rühren in 4500 ml Wasser von 20° C eingetragen. Nach 30 Minuten wird die Suspension unter weiterem Rühren auf 60—70° C erwärmt. Nach einer Gesamtzeit von 1,5 Stunden wird der verbleibende Rückstand filtriert, mit Wasser nachgewaschen und anschließend bei 120° C/2 mbar getrocknet. Die Auswaage ergibt 25 g, ihre Analyse 0,525 g Rhodium. Dieser Rückstand wird zur Entfernung von undestillierbaren organischen Verunreinigungen im Soxhlet-Apparat mit 500 ml Diethylether beim Siedepunkt des Ethers extrahiert. Nach drei- bis fünfstündiger Extraktion und anschließender Trocknung wird ein etherunlöslicher Rückstand von 6 g erhalten. Der Rhodiumgehalt beträgt 0,520 g, entsprechend einer Ausbeute von 99%. Der etherunlösliche Rückstand enthält nahezu den gesamten in den Reinigungsprozeß eingesetzten Rhodiumcarbonylkomplex, der wieder in die Reaktion zurückgeführt wird.

Die Diethyletherphase wird eingedampft, wobei 19 g undestillierbare organische Verunreinigungen (= 7,6 Gew.-%, ber. auf 250 g Katalysatorlösung) im Kolben verbleiben. Laut Analyse besitzt dieser Rückstand noch einen Rhodiumgehalt von 0,026 Gew.-%. Die abdestillierte Etherphase wird zur erneuten Extraktion benutzt.

Die bei der Filtration anfallende Wasserphase wird zur Rückgewinnung des organischen Promotors unter vermindertem Druck aufgearbeitet. Nach Abdampfen des Wassers werden 160,5 g N,N-Dimethylimidazoliumjodid entsprechend einer Ausbeute von 99,7% erhalten. Die N/J-Analyse mit 12,5 Gew.-% N und 56,7 Gew.-% Jod sowie die IR-Analyse beweisen den hohen Reinheitsgrad des zurückgewonnenen organischen Promotorsalzes. Das organische Promotorsalz wird in die Reaktion und das abdestillierte Wasser einer erneuten Extraktion zugeführt.

## Beispiel 4

Dem aus Palladiumcarbonylkomplex, Tri-n-butylmethylphosphoniumjodid als organischem Promotor, undestillierbaren organischen Verunreinigungen, Essigsäure, Essigsäureanhydrid und Ethylidendiacetat bestehenden Katalysatorkreislauf der Methylacetatcarbonylierung werden 250 g Katalysatorlösung entnommen und unter vermindertem Druck von 2 mbar sowie einer Blasentemperatur bis zu 120° C von den destillierbaren Anteilen befreit. Dabei werden 80 g (32 Gew.-%) Destillat (51,9 Gew.-% Essigsäure, 10 Gew.-% Essigsäureanhydrid und 38,1 Gew.-% Ethylidendiacetat) und 170 g Destillationsrückstand mit 0,85 g Palladium und 152 g Tri-n-butylmethylphosphoniumjodid (= 2 Gew.-% Pdcarbonylkomplex und 60,8 Gew.-% TBMPJ, ber. auf 250 g Katalysatorlösung) erhalten. Der Destillationsrückstand wird zerkleinert und anschließend unter Rühren in 5000 ml Wasser von 20° C eingetragen. Nach 30 Minuten wird die Suspension unter weiterem Rühren auf 65° C erwärmt. Nach einer Gesamtzeit von 1,5 h wird der Rückstand filtriert, mit Wasser nachgewaschen und anschließend bei 120° C/2 mbar getrocknet. Die Auswaage beträgt 18 g mit einem Gehalt von 0,846 g Palladium. Dieser wasserunlösliche Rückstand wird zur Entfernung der undestillierbaren organischen Verunreinigungen im Soxhlet-Apparat mit 500 ml Diisopropylether bei Siedetemperatur des Ethers extrahiert. Nach 4 stündiger Extraktion und anschließender Trocknung wird ein etherunlöslicher Rückstand von 7 g erhalten. Sein Palladiumgehalt beträgt 0,84 g entsprechend einer Ausbeute von 98,8%. Der etherunlösliche Rückstand enthält nahezu den gesamten in den Reinigungsprozeß eingesetzten Palladiumcarbonylkomplex, der als solcher wieder in die Reaktion eingesetzt wird.

Die Diisopropyletherphase wird eingedampft, wobei 11 g undestillierbare organische Verunreinigungen (= 4,4 Gew.-%, ber. auf 250 g Katalysatorlösung) mit einem Pd-Gehalt von 0,03 Gew.-% im Kolben verbleiben. Der abdestillierte Ether wird zur erneuten Extraktion benutzt.

Die bei der Filtration anfallende Wasserphase wird zur Rückgewinnung des organischen Promotors unter vermindertem Druck aufgearbeitet. Nach Abdampfen des Wassers werden 151 g Tri-n-butylmethylphosphoniumjodid entsprechend einer Ausbeute von 99,3% erhalten. Das organische Promotorsalz wird der Carbonylierungsreaktion und das abdestillierte Wasser einer erneuten Extraktion zugeführt.

**Patentansprüche**

1. Verfahren zur Reinigung und Rückgewinnung der bei der Carbonylierung von Methylacetat und/oder Dimethylether anfallenden, verunreinigten Katalysatorlösung, welche Carbonylkomplexe von Edelmetallen der Gruppe VIII des Periodensystems der Elemente, quaternäre heterocyclische aromatische Stickstoffverbindungen oder quaternäre Organophosphorverbindungen als organische Promotoren, undestillierbare organische Verunreinigungen sowie Essigsäure, Essigsäureanhydrid und Ethylidendiacetat enthält, dadurch gekennzeichnet, daß man die flüchtigen Bestandteile aus der Katalysatorlösung abdestilliert und den verbleibenden festen Destillationsrückstand mit Wasser behandelt, wobei der Edelmetall-Carbonyl-Komplex zusammen mit den organischen Verunreinigungen ausgefällt wird, während der organische Promotor in Lösung geht; daß man den ausgefällten verunreinigten Edelmetall-Carbonyl-Komplex abfiltriert und durch Extraktion mit aliphatischen Ethern von den organischen Verunreinigungen befreit; daß man den organischen Promotor durch Abdampfen des Wassers oder durch Extraktion mit Halogenkohlenwasserstoffen und Abdampfen derselben zurückgewinnt und daß man den gereinigten Edelmetall-Carbonyl-Komplex sowie den zurückgewonnenen organischen Promotor in die Carbonylierungsreaktion zurückführt.

2. Verfahren nach Anspruch 1, dadurch gekennzeichnet, daß man die Behandlung des Destillationsrückstands mit Wasser bei 40—80° C vornimmt.

3. Verfahren nach einem der vorhergehenden Ansprüche, dadurch gekennzeichnet, daß man je Gewichtsteil Destillationsrückstand 10 bis 100 Gewichtsteile Wasser einsetzt.

4. Verfahren nach einem der vorhergehenden Ansprüche, dadurch gekennzeichnet, daß man den abfiltrierten Edelmetall-Carbonyl-Komplex mit Diethyl- oder Diisopropylether extrahiert.

5. Verfahren nach einem der vorhergehenden Ansprüche, dadurch gekennzeichnet, daß man quaternäre Organophosphorverbindungen aus ihrer wäßrigen Lösung durch Extraktion mit Methylenchlorid oder Chloroform zurückgewinnt.

6. Verfahren nach einem der vorhergehenden Ansprüche, dadurch gekennzeichnet, daß die Katalysatorlösung zusätzlich Verbindungen carbonylbildender Nicht-Edelmetalle als anorganische Promotoren enthält, welche bei der Behandlung des festen Destillationsrückstandes mit Wasser

a) zusammen mit dem organisch verunreinigten Edelmetall-Carbonyl-Komplex ausgefällt, abfiltriert, durch Extraktion mit Ether gereinigt und zusammen mit dem Edelmetall-Carbonyl-Komplex in die Carbonylierungsreaktion zurückgeführt werden und/oder

b) zusammen mit dem organischen Promotor in Lösung gehen und durch Abdampfen des Wassers oder Extraktion mit Halogenkohlenwasserstoffen und Abdampfen derselben zurückgewonnen und zusammen mit dem organischen Promotor in die Carbonylierungsreaktion zurückgeführt werden.

**Claims**

1. Process for purifying and recovering the contaminated catalyst solution obtained in the carbonylation of methyl acetate and/or dimethylether, containing carbonyl complexes of noble metals of group VIII of the Periodic System of the elements, quaternary heterocyclic aromatic nitrogen compounds or quaternary organophosphorus compounds as organic promoters, undistillable organic contaminants as well as acetic acid, acetic anhydride and ethylidene diacetate, which comprises: distillatively freeing the catalyst solution from its volatile constituents and water-treating the remaining solid distillation residue, the noble metal/carbonyl-complex being precipitated together with the organic contaminants with the organic promoter becoming dissolved; removing the precipitated contaminated noble metal/carbonylcomplex by filtration and freeing it from the organic contaminants by extraction with aliphatic ethers; recovering the organic promotor by evaporating the water or extracting it with halogenated hydrocarbons and evaporating these latter; and recycling the purified noble metal/carbonyl-complex and the recovered organic promoter into the carbonylation reaction.

2. Process as claimed in claim 1, wherein the distillation residue is water-treated at 40 to 80°C.

3. Process as claimed in any of the preceding claims, wherein 10 to 100 parts by weight water are used per part by weight distillation residue.

4. Process as claimed in any of the preceding claims, wherein the noble metal/carbonyl-complex filtered off is extracted with diethyl- or diisopropylether.

5. Process as claimed in any of the preceding claims, wherein the quaternary organophosphorus compounds are recovered from their aqueous solution by extraction with methylene chloride or chloroform.

6. Process as claimed in any of the preceding claims, wherein the catalyst solution additionally contains carbonyl-yielding non noble metals as inorganic promoters which, during the water-treatment of the solid distillation residue, are

a)  precipitated together with the noble metal/carbonyl-complex contaminated with organic impurities, filtered off, purified by extraction with an ether and recycled together with the noble metal/carbonyl-complex to the carbonylation reaction, and/or
b)  dissolved together with the organic promoter and recovered by evaporation of the water or by extraction with halogenated hydrocarbons and evaporation of these latter, and recycled together with the organic promoter to the carbonylation reaction.

## Revendications

1. Procédé de purification et de récupération de la solution catalytique contaminée obtenue dans la carbonylation d'acétate de méthyle et/ou d'éther diméthylique et contenant des complexes carbonylés de métaux nobles appartenant au groupe VIII de la Classification Périodique des éléments, des composés azotés aromatiques hétérocycliques quaternaires ou des composés organophosphorés quaternaires comme activeurs organiques, des impuretés organiques non distillables ainsi que de l'acide acétique, de l'anhydride acétique et du diacétate d'éthylidène, caractérisé en ce que l'on sépare par distillation les constituants volatils de la solution catalytique et on traite par l'eau le résidu de distillation solide restant, le complexe métal noble-carbonyle étant précipité conjointement avec les impuretés organiques tandis que l'activeur organique est dissous; en ce que l'on sépare par filtration le complexe métal noble-carbonyle contaminé précipité et on le débarrasse des impuretés organiques par extraction par des éthers aliphatiques; en ce que l'on récupère l'activeur organique par évaporation de l'eau ou par extraction par des hydrocarbures halogénés et évaporation de ceux-ci; et en ce que l'on recycle dans la réaction de carbonylation le complexe métal noble-carbonyle purifié ainsi que l'activeur organique récupéré.

2. Procédé selon la revendication 1, caractérisé en ce que l'on effectue le traitement par l'eau du résidu de distillation à 40—80°C.

3. Procédé selon l'une des revendications précédentes, caractérisé en ce que l'on utilise 10—100 parties en poids d'eau par partie en poids de résidu de distillation.

4. Procédé selon l'une des revendications précédentes, caractérisé en ce que l'on extrait par l'éther diéthylique ou diisopropylique le complexe métal noble-carbonyle filtré.

5. Procédé selon l'une des revendications précédentes, caractérisé en ce que l'on récupère les composés organophosphorés quaternaires de leur solution aqueuse par extration par le chlorure de méthylène ou le chloroforme.

6. Procédé selon l'une des revendications précédentes, caractérisé en ce que la solution catalytique contient en outre des composés de métaux non nobles formant des carbonyles comme activeurs inorganiques qui lors du traitement à l'eau du résidu de distillation solides sont

a)  précipités conjointement avec le complexe métal noble-carbonyle contaminé par des impuretés organiques, filtrés et purifiés par extraction par un éther et recyclés dans la réaction de carbonylation conjointement avec le complexe métal noble-carbonyle et/ou
b)  dissous conjointement avec l'activeur organique, récupérés par évaporation de l'eau ou extraction par des hydrocarbures halogénés et évaporation de ceux-ci et recyclés dans la réaction de carbonylation conjointement avec l'activeur organique.